# EUROPÄISCHE PATENTSCHRIFT

(11) **EP 1 183 004 B1**
(45) Veröffentlichungstag und Bekanntmachung des Hinweises auf die Patenterteilung: **15.09.2004**
(21) Anmeldenummer: 00943670.0
(22) Anmeldetag: 26.05.2000
(51) Int. Cl.: A61K 7/00

(54) **PARFÜMKOMPOSITION MIT DUFTSEQUENZ**
PERFUME COMPOSITIONS WITH A SCENT SEQUENCE
COMPOSITIONS DE PARFUM A SEQUENCE PARFUMEE

(30) Priorität: 31.05.1999 DE 19925971
(43) Veröffentlichungstag der Anmeldung: 06.03.2002
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: PCT/DE2000/001783
(87) Internationale Veröffentlichungsnummer: WO 2000/072804

(56) Entgegenhaltungen:
- WO-A-95/16432
- DE-A- 19 933 452
- US-A- 5 508 259
- PATENT ABSTRACTS OF JAPAN vol. 015, no. 154 (C-0825), 18. April 1991 (1991-04-18) & JP 03 032673 A (LION CORP), 13. Februar 1991 (1991-02-13)

## Beschreibung

Die Erfindung betrifft Parfümkompositionen, bei denen unterschiedliche Duftnoten nacheinander freigesetzt werden (Duftsequenz).

Es ist bekannt, daß Parfümrezepturen mit unterschiedlicher Flüchtigkeit nach dem öffnen einer Parfümflasche eine sog. Kopfnote freisetzen, danach eine Herznote und schließlich eine Fondnote. Die Kopfnote wird allgemein durch niedrigsiedende Alkohole wie Ethanol bestimmt, und eine verlängerte Fondnote ist beispielsweise durch schwerer flüchtige öle oder Bindung an einen Träger unter Mithilfe hochsiedender Lösungsmittel möglich.

Um die Flüchtigkeit von Parfümzusammensetzungen zu beeinflussen und eine möglichst lange Wirksamkeit auf der Haut des Trägers zu erreichen, sind u.a. Parfüme in Kapseln eingeschlossen worden. So ist aus der EP 838216 eine Verkapselung mit high-bloom Gelatinekapseln bekannt, bei der der Duftstoff zusammen mit einem flüchtigen Lösungsmittel und einem nichtflüchtigen Co-Lösungsmittel eingekapselt vorliegt. Weiterhin sind Parfümzusammensetzungen mit Cyclodextrinen bekannt (z.B. EP 0013688) oder mit Cyclodextrinen in einem wäßrigen Träger, Feuchthaltemitteln und oberflächenaktiven Mitteln (z.B. WO 98/56341).

Der Erfindung liegt die Aufgabe zugrunde, eine Parfümzusammensetzung bereitzustellen, bei der beim Auftragen auf die Haut verschiedene Duftnoten nacheinander, jedoch nahezu vollständig isoliert voneinander frei werden.

Erfindungsgemäß ist die Parfümkomposition mit Duftsequenz, gekennzeichnet durch
a) wenigstens eine erste Parfümzusammensetzung, die aus wenigstens einem ersten und einem zweiten Duftstoff in Abwesenheit von Alkohol besteht, wobei die Duftstoffe in Mikrokapseln eingeschlossen sind, und
b) wenigstens eine zweite Parfümzusammensetzung, die aus wenigstens einem dritten und einem vierten Duftstoff in Abwesenheit von Alkohol besteht, wobei die Duftstoffe in Mikrokapseln eingeschlossen sind, und
c) ein kosmetisches Gel, in dem die erste und die zweite Parfümkomposition verteilt vorliegen,
wobei die Mikrokapseln aus einem flüssigen Suspensionsmedium und wenigstens einer darin nicht mischbaren Phase durch Emulsion oder Dispersion gebildet sind und eine Wandstärke haben, die von der ersten Parfümzusammensetzung zur zweiten Parfümzusammensetzung und gegebenenfalls jeder weiteren Parfümzusammensetzung ansteigend ist, und
daß die ersten, zweiten, dritten und vierten Duftstoffe eine unterschiedliche Flüchtigkeit haben, die vom ersten zum vierten und gegebenenfalls jedem weiteren Duftstoff ansteigend ist.

Eine vorteilhafte Ausführungsform der Erfindung besteht darin, daß die Mikrokapseln der ersten Parfümzusammensetzung oder der zweiten Parfümzusammensetzung oder beide neben den Duftstoffen lamellare Flüssigkristalle oder Flüssigkristallgemische enthalten.

Die Herstellung der Mikrokapseln erfolgt nach üblichen Verfahren, bei denen eine Flüssigkeit als Suspensionsmedium für die Mikrokapseln verwendet wird, und die Emulgierung/Dispersion von zwei oder mehreren nicht miteinander mischbaren Phasen erfolgt. Zu diesen Verfahren gehören die Grenzflächenpolymerisation, in situ-Polymerisation, Lösungsmittelverdampfung, Gelatinierung, Druckextrudierung, Polymer-Polymer-Inkompatibilität und einfache und komplexe Koazervation. Die drei letztgenannten Verfahren sind bevorzugt.

Bei der Koazervation lagern sich die einzelnen Tröpfchen des Wandpolymeren an der Oberflächen eines Kernmaterials ab, wobei es wesentlich ist, daß der Kern in dem Lösungsmittel des Polymermaterials nicht löslich ist, und das Wandpolymere sich kaum in dem Kernmaterial verteilen kann. Nach vollständiger Bedekkung der Oberfläche des Kernmaterials mit den Mikrotröpfchen und Koaleszenz bilden die Koazervate eine Wand um das Kernmaterial. Diese Wand kann gegebenenfalls noch gehärtet werden. [siehe zur Koazervation z.B. Hallcrest, Handbook of Thermochrome Lipid Crystal Technology (1991) S. 11-12].

Bei Wahl von Flüssigkristallen als Kernmaterial kann der oben geschilderte Vorgang auch in der Weise erfolgen, daß die lamellaren Flüssigkristalle mit den entsprechenden Duftstoffen in Kontakt gebracht und zu Koazervaten umgewandelt werden. Nach den bekannten Verfahren können dabei unterschiedliche Wandstärken erreicht werden.
Die gebildeten Kapseln können einen Durchmesser im Bereich von 5-10 mm bis zu 2000 µm haben. Die Wandstärken liegen bei Durchmessern unter 500 µm im Bereich von 5 bis 20 µm.

Bei einer bevorzugten Ausführungsform liegen die Wandstärken der Mikrokapseln im Bereich von 100 bis 2000 nm.

Als Flüssigkristalle sind sowohl cholesterische Substanzen wie Cholesterol, Cholesterolester, Phytosterole insbesondere B-Sitosterol und Camposterol, Phytosterolester, Dihydrosterolester, nichtspezifische sterolderivate oder auch Gemische von Cholesterol mit 2-Acetaminoalkan-1,3-diolen und 2-Acylaminoalkan-1,3-diolen, als auch chiral nematische Substanzen wie z.B. (2-Methylbutyl)phenyl-4-alkyl(oxy)benzoate einsetzbar.

Als Duftstoffe in den erfindungsgemäßen Parfümkompositionen werden beispielhaft genannt: Ambre, Anekole C₁₀H₁₂O, Angelique Rook Qil, Artemisia oil, Basil oil, Rose oil, Lavel Oil, Bay Oil, Benzaldehyde C₇H₆O, Bergamot oil, Benzyl Acetate C₉H₁₀O₂, Camphre C₁₀H₁₆O, Calmusoil, Carotte oil, chamomille Oil, Citronella Oil, Couramine C₉H₆O₂, cypren oil, Dihydromyrcenol C₁₀H₂₀O, Jasmin, Mimosa, Muscone C₁₆H₃₀O, Narcissus, orange Oil, Rose oxide C₁₀H₁₈O, Sanddorn wood oil, Vanille C₈H₈O₃. Eine Vielzahl weiterer Duftstoffe sind einsetzbar, insbesondere Varianten einzelner Duftstoffe mit ähnlichen Geruchsnuancen.

Für die Erfindung wesentlich ist, daß jeweils wenigstens zwei Duftstoffe eine von mehreren Parfümzusammensetzungen innerhalb der erfindungsgemäßen Parfümkomposition bilden und daß kein leicht flüchtiges Lösungsmittel, d.h. kein Alkohol diesen Parfümzusammensetzungen zugesetzt ist.

Die Wandstärke der Mikrokapseln ist bei den einzelnen Parfümzusammensetzungen unterschiedlich und wird von der ersten Parfümzusammensetzung zur zweiten Parfümzusammensetzung und gegebenenfalls jeder weiteren Parfümzusammensetzung ansteigend gestaltet. Dadurch kann ein Aufbrechen der Kapselwand der Mikrokapsel zu unterschiedlichen Zeiten erfolgen.

So können z.B. Kapseln mit der ersten Parfümzusammensetzung eine Wandstärke von 100 bis 200 nm haben, die Kapseln der zweiten Parfümzusammensetzung eine Wandstärke von 400 bis 500 nm und die Kapseln der dritten Parfümzusammensetzung eine Wandstärke von >500 nm.

Mit Hilfe der Erfindung ist es möglich, unterschiedliche Duftnoten, bestehend aus wenigstens zwei verschiedenen Duftstoffen, in mikroverkapselter Form auf die Haut aufzutragen, durch das leichte anfängliche Aufstreichen des Gels auf die Haut die Kapselwand der Kapsel mit einer ersten Parfümzusammensetzung zu zerstören und damit das Gemisch des ersten und zweiten Duftstoffes als "Kopfnote" des Parfüms freizusetzen.
Zu einem etwas späteren Zeitpunkt wird eine zweite Duftnote in Form des Gemisches des wenigstens dritten und vierten Duftstoffes freigesetzt, wobei zwischen beiden Duftstoffen keine wesentliche Mischduftphase auftritt.

Gegebenenfalls wird durch noch eine dritte oder danach weitere Duftnote in Form eines Gemisches eines wenigstens fünften und sechsten und gegebenenfalls weiterer Duftstoffe (Gemische) freigesetzt, jeweils ohne wesentliche Mischduftphase.

Es ist bisher noch nicht geklärt, wodurch das Freisetzen der weiteren Duftstoffe ohne sichtbaren äußeren Einfluß ermöglicht wird und worauf der Wegfall einer Mischduftphase zurückzuführen ist. Eine Wechselwirkung mit Hautproteinen und der Einfluß der Hauttemperatur kann vermutet werden.

Unter "ohne wesentliche Mischduftphase" im Sinne der Erfindung wird verstanden, daß ein Gemisch von zwei Duftnoten kaum wahrnehmbar ist und wenn überhaupt, wenige Sekunden (z.B. 2-8 Sekunden) auftritt.

Durch die erfindungsgemäße Kombination wird ein von der Anwenderin/dem Anwender gewünschte Duftsequenz kreiert, die bisher in dieser Form nicht hervorzubringen war. Zwar konnte bisher entsprechend der Hauthaftung und unterschiedlichen Lösungsmitteln mit variierten Siedepunkten sowie gegebenenfalls weiteren Zusatzstoffen auch eine Duftfolge hergestellt werden, jedoch war dies erstens immer mit der Anwesenheit von Lösungsmitteln wie z.B. Alkoholen verbunden, und es entstand zweitens stets eine länger dauernde und kaum beeinflußbare Mischduftphase, in der keine klare Duftnote herausfindbar war.

Weiterhin ermöglicht die Erfindung durch den Einsatz von Flüssigkristallen mit lamellarer Phase eine entsprechende länger dauernde Bindung der Duftstoffe daran und eine insgesamt verzögerte Freisetzung der Düfte.

Zusätzlich ermöglichen temperaturempfindliche und thermochrome Flüssigkristalle besonders attraktive Farbeffekte der Mikrokapseln in dem Gel. Dies kann noch verstärkt werden durch Viskositätseinstellung des Gels auf höhere Viskositäten, z.B. oberhalb 50000 Pa·s; in der Weise, daß die unterschiedlichen Mikrokapseln punktuell, spiralig, an diskreten Stellen im Gel, schichtweise oder in sonstiger Verteilung im Gel abgelagert und entsprechend auch an diesen Stellen gehalten werden können. Durch intensives Schütteln oder durch Verteilung in einem weniger viskosen Gel kann auch eine gleichmäßige Verteilung der Mikrokapseln erreicht werden.

Bei einer bevorzugten Parfümkomposition der Erfindung sind drei Parfümzusammensetzungen eingeschlossen. Die erste Parfümzusammensetzung enthält zwei unterschiedliche Orangenduftstoffe, die zweite Parfümzusammensetzung enthält zwei unterschiedliche Vanilleduftstoffe, und die dritte Parfümzusammensetzung enthält zwei unterschiedliche Rosenduftstoffe.

Wie bereits oben ausgeführt ist eine vorteilhafte Ausführungsform der Erfindung darin zu sehen, daß Wandstärke der Mikrokapseln und die Flüchtigkeit der Duftstoffe so gewählt werden, daß nach Auftragen der Parfümkomposition auf die Haut zuerst die Duftstoffe der ersten Parfümzusammensetzung frei werden und danach ohne eine wesentliche Mischduftphase die Düfte der zweiten Parfümzusammensetzung frei werden und danach die Duftstoffe der dritten und gegebenenfalls weiterer Parfümzusammensetzungen.

Die Einlagerung der Parfümzusammensetzungen kann in bekannten kosmetischen Gelen erfolgen. Dazu gehören Carbomer, Xanthangummi, Carrageenan, Akaziengummi, Guargummi, Agar-Agar, Alginate und Tylosen, Carboxymethylcellulose, Hydroxyethylcellulose, bestimmte Polyacrylate, Polyvinylalkohol, Polyvinylpyrrolidon, Silicongele.

Die Erfindung soll nachstehend durch Beispiele näher erläutert werden. Alle Prozentangaben sind Gewichtsprozente, wenn nichts anderes angegeben ist.

### Beispiel 1

Flüssigkristalle des Typs Cholesteryl 2,4 Dichloro Benzoate, Cholesteryl Oleyl carbonate, Cholesteryl Nonanoate und Cholesteryl Chloride wurden zusammen mit einem ersten Duftstoffgemisch aus zwei unterschiedlichen Orangeduftstoffen (Duftstoffliquid 1) mit einer Konzentration von 8 Gew-% in Parfümöl in Kontakt gebracht und auf übliche Weise zu einem Koazervat umgewandelt. Dabei betrug die Wandstärke der gebildeten Mikrokapseln etwa 120 bis 150 nm.

Danach wurden die Flüssigkristalle Cholesteryl Oleyl Carbonate, Cholesteryl Nonanoate und Cholesteryl Chloride mit einem zweiten Duftstoffgemisch aus zwei unterschiedlichen Vanilleduftstoffen (Duftstoffliquid 2) mit einer Konzentration von 8 Gew-% in Öl in Kontakt gebracht und auf übliche Weise zu einem Koazervat umgewandelt. Dabei betrug die Wandstärke der gebildeten Mikrokapseln etwa 300 bis 450 nm.

Danach wurden die Flüssigkristalle Cholesteryl Oleyl Carbonate, Cholesteryl Nonanoate und Cholesteryl Chloride mit einem dritten Duftstoffgemisch aus zwei unterschiedlichen Rosenduftstoffen (Duftstoffliquid 3) mit einer Konzentration von 8 Gew-% in Parfümöl in Kontakt gebracht und auf übliche weise zu einem Koazervat umgewandelt. Dabei betrug die Wandstärke der gebildeten Mikrokapseln etwa >500 nm.

Nacheinander wurden die drei Duftstoffe bei einer Temperatur im Bereich von 15 bis 45 °C und unter vorsichtiger Verteilung oder durch Einspritzen in ein Silicongel eingebracht, so daß sich folgende Zusammensetzung ergab (in Gew-%):

| | |
|---|---|
| Siliconpulver | 3 |
| Siliconöl | ad 100 |
| Duftstoffliquid 1 | 2 |
| Duftstoffliquid 2 | 2 |
| Duftstoffliquid 3 | 2 |

Die einzelnen Tröpfchen der Parfümkomposition waren durch Farbunterschiede deutlich erkennbar und hatten somit zusätzlich einen attraktiven optischen Effekt.

## Patentansprüche

1. Parfümkomposition mit Duftsequenz, **gekennzeichnet durch**
a) wenigstens eine erste Parfümzusammensetzung, die aus wenigstens einem ersten und einem zweiten Duftstoff in Abwesenheit von Alkohol besteht, wobei die Duftstoffe in Mikrokapseln eingeschlossen sind, und
b) wenigstens eine zweite Parfümzusammeasetzung, die aus wenigstens einem dritten und einem vierten Duftstoff in Abwesenheit von Alkohol besteht, wobei die Duftstoffe in Mikrokapseln eingeschlossen sind, und
c) ein kosmetisches Gel, in dem die erste und die zweite Parfümkomposition verteilt vorliegen,
wobei die Mikrokapseln aus einem flüssigen Suspensionsmedium und wenigstens einer darin nicht mischbaren Phase **durch** Emulsion oder Dispersion gebildet sind und eine Wandstärke haben, die von der ersten Parfümzusammensetzung zur zweiten Parfümzusammensetzung und gegebenenfalls jeder weiteren Parfümzusammensetzung ansteigend ist, und
daß die ersten, zweiten, dritten und vierten Duftstoffe eine unterschiedliche Flüchtigkeit haben, die vom ersten zum vierten und gegebenenfalls jedem weiteren Duftstoff ansteigend ist.

2. Parfümkomposition nach Anspruch 1, **dadurch gekennzeichnet, daß** die Mikrokapseln der ersten Parfümzusammensetzung oder der zweiten Parfümzusammensetzung oder beide durch Koazervation gebildet sind und neben den Duftstoffen lamellare Flüssigkristalle oder Flüssigkristallgemische enthalten.

3. Parfümkomposition nach Anspruch 1, **dadurch gekennzeichnet, daß** die Wandstärken der Mikrokapseln im Bereich von 100 bis 2000 nm liegen.

4. Parfümkomposition nach einem der Ansprüche 1 bis 3, **dadurch gekennzeichnet, daß** drei unterschiedliche Parfümzusammensetzungen eingeschlossen sind, und die erste Parfümzusammensetzung zwei orangendnftstoffe enthält, die zweite Parfümzusammensetzung zwei Vanilleduftstoffe enthält und die dritte Parfümzusammensetzung zwei Rosenduftstoffe enthält.

5. Parfümkomposition nach einem der Ansprüche 1 bis 4, **dadurch gekennzeichnet, daß** die Mikrokapseln und die Duftstoffe so gewählt werden, daß nach Auftragen der Parfümkomposition auf die Haut zuerst die Duftstoffe der ersten Parfümzusammensetzung frei werden und zeitlich danach ohne eine wesentliche Mischduftphase die Düfte der zweiten Parfümzusammensetzung frei werden.

6. Verwendung einer Parfümkomposition mit Duftsequenz, **dadurch gekennzeichnet, daß** man eine Parfümkomposition nach Anspruch 1 auf die Baut aufbringt und ohne äußere Einwirkung und ohne eine wesentliche Mischduftphase die einzelnen Parfümzusammensetzungen frei werden läßt.

## Claims

1. Perfume composition with a scent sequence, comprising
a) at least a first perfume ingredient consisting of at least a first and a second aromatic substance in the absence of alcohol, wherein the aromatic substances are contained in microcapsules, and
b) at least a second perfume ingredient consisting of at least a third and a fourth aromatic substance in the absence of alcohol, wherein the aromatic substances are contained in microcapsules, and
c) a cosmetic gel in which the first and the second perfume ingredients are distributed,
where the microcapsules are formed by emulsion or dispersion from a liquid suspension medium and at least one phase which is not miscible with the suspension medium,
said microcapsules have a wall thickness which increases from the first perfume ingredient to the second perfume ingredient and, if applicable, to any further perfume ingredient, and
wherein the first, second, third and fourth aromatic substances have different volatilities which increases from the first to the fourth and, if applicable, any further aromatic substance.

2. Perfume composition according to claim 1, wherein the microcapsules of the first perfume ingredient or the second perfume ingredient or both contain, apart from the aromatic substances, lamellar liquid crystals or liquid crystal mixtures.

3. Perfume composition according to claim 1, wherein the wall thicknesses of the microcapsules range from 100 to 2000 nm.

4. Perfume composition according to any of claims 1 to 3, comprising three different perfume ingredients, wherein the first perfume ingredient comprises two orange scent substances, the second perfume ingredient two vanilla scent substances, and the third perfume ingredient two rose scent substances.

5. Perfume composition according to any of claims 1 to 4, wherein the microcapsules and the aromatic substances are selected so that, after applying the perfume composition onto the skin, at first the aromatic substances of the first perfume ingredient are released and at a later time, without an essential mixed scent phase, the aromatic substances of the second perfume ingredient are released.

6. Use of a perfume composition with a scent sequence, wherein a perfume composition according to claim 1 is applied onto the skin to allow, without external action and without an essential mixed scent phase, the individual perfume ingredients to be released.

## Revendications

1. Composition de parfum à séquence parfumée, **caractérisé par**
a) au moins un premier composé de parfum, qui est constitué d'au moins une première et une deuxième substance odorante en présence d'alcool, les substances odorantes étant incluses dans des microcapsules, et
b) au moins un second composé de parfum qui est constitué d'au moins une troisième et une quatrième substance odorante en présence d'alcool, les substances odorantes étant incluses dans des microcapsules, et
c) un gel cosmétique dans lequel le premier et le second composé de parfum sont répartis, les microcapsules étant formées d'un premier milieu de suspension liquide et d'au moins une phase ne pouvant s'y mélanger par émulsion ou dispersion et ayant une épaisseur de paroi croissante du premier composé de parfum vers le deuxième composé de parfum et le cas échéant vers chaque composé de parfum suivant et
**caractérisée en ce que** les premières, deuxième, troisième et quatrième substances odorantes présentent une volatilité différente qui est croissante de la première à la quatrième substance odorante et le cas échéant à chaque substance odorante suivante.

2. Composition de parfum selon la revendication 1, **caractérisée en ce que** les microcapsules du premier composé de parfum ou du deuxième composé de parfum ou des deux sont formées par coacervation et contiennent, outre les substances odorantes, des cristaux liquides lamellaires ou des mélanges de cristaux liquides.

3. Composition de parfum selon la revendication 1, **caractérisée en ce que** l'épaisseur des parois des microcapsules est comprise entre 100 et 2 000 nm.

4. Composition de parfum selon l'une des revendications 1 à 3, **caractérisée en ce que** trois composés de parfum différents sont inclus et le premier composé de parfum contient deux substances odorantes d'orange, le deuxième composé de parfum contient deux substances odorantes de vanille et le troisième composé de parfum contient deux substances odorantes de rose.

5. Composition de parfum selon l'une des revendications 1 à 4, **caractérisée en ce que** les microcapsules et les substances odorantes sont choisies de telle sorte qu'après application de la composition de parfum sur la peau d'abord les substances odorantes du premier composé de parfum soient libérés et ensuite sans phase de mélange de senteur importante les senteurs du deuxième composé de parfum soient libérées.

6. Utilisation de la composition de parfum à séquence parfumée, **caractérisée en ce qu'**on applique sur la peau une composition de parfum selon la revendication 1 et **en ce que** sont libérés, sans action externe et sans phase de mélange de senteur importante, les composés de parfum individuels.
